# EUROPEAN PATENT APPLICATION

(11) **EP 4 477 079 A1**
(43) Date of publication of application: **18.12.2024**
(21) Application number: 23382580.1
(22) Date of filing: 12.06.2023
(51) Int. Cl.: A01K 67/027, C12N 5/079

(54) **METHOD FOR GENERATING A HUMANIZED ANIMAL MODEL OF A MENTAL DISORDER FROM THE HUMAN OLFACTORY NEUROEPITHELIUM**

(71) Applicant: Universidad de Cádiz, 11002 Cádiz (ES); Consorcio Centro de Investigación Biomédica en Red, 28029 Madrid (ES); Servicio Andaluz de Salud, 41071 Sevilla (ES); Fundació Institut Hospital del Mar d'Investigacions Mèdiques, 08003 Barcelona (ES); Administración General de la Comunidad Autónoma de Euskadi, 01010 Vitoria-Gasteiz, Álava (ES); Fundació de Recerca Clínic Barcelona-Institut d'Investigacions Biomèdiques August Pi I Sunyer, 08036 Barcelona (ES)
(72) Inventor: Berrocoso Domínguez, Esther María, 11002 Cádiz (ES); Hidalgo Figueroa, María, 11002 Cádiz (ES); Suárez Pereira, Irene, 11002 Madrid (ES); Delgado Sequera, Alejandra Cristina, 11002 Cádiz (ES); Romero López-Alberca, Cristina, 11002 Cádiz (ES); Durán Ruiz, María del Carmen, 11002 Cádiz (ES); Pérez Revuelta, José Ildefonso, 11002 Cádiz (ES); González Saiz, Francisco Manuel, 11002 Cádiz (ES); Pérez Sola, Víctor, 08003 Barcelona (ES); González-Pinto Arrillaga, Ana, 01010 Vitoria-Gasteiz (Álava) (ES); Vieta Pascual, Eduard, 08036 Barcelona (ES); García Mompó, Clara, 41071 Sevilla (ES)
(74) Representative: Hoffmann Eitle

(57) **Abstract**

The present invention refers to a method for generating a humanized animal model of a mental disorder. The model is generated by implanting neural precursors from the olfactory neuroepithelium of patients with said mental disorder in the brain of adult animals. The model can be used for the study of the disease, as well as for the identification and evaluation of the efficacy of potential treatments against the disease.

## Description

### FIELD OF THE INVENTION

The present invention belongs to the field of animal models in psychiatry and psychopharmacology. Particularly, it refers to a method for generating an animal model that recapitulates the symptomatology present in a donor patient suffering from a mental disorder.

### STATE OF THE ART

A mental disorder, also referred to as a mental illness or psychiatric disorder, is a behavioral or mental pattern that causes significant distress or impairment of personal functioning.

The etiology of a mental disorder is typically based on a combination of genetic and environmental factors. Family, twin, and adoption studies have firmly established the roles of both genes and environment in mental disorders. It remains difficult, however, to find genes for these disorders, and to characterize the particular environmental circumstances under which psychopathology emerges. The reason for this difficulty lies in the complex nature of mental disorders. Many disorders probably result from the combined action of multiple genes of small effect together with a variety of environmental factors. In addition, genetic and environmental factors interact with each other in complex ways to influence phenotype.

Recent advances in the identification of genes associated to mental disease have led to the development of animal models for multiple mental diseases, which were designed with the goal attaining translational utility to ultimately develop novel treatments. However, modelling mental disease is still a challenge nowadays. On the one hand, *in vitro* models derived from biological samples from patients suffering from mental disorders do not recapitulate the structural and cellular complexity of the human brain, which may be crucial to model mental disease. On the other, current *in vivo* animal models of mental disorders cannot reproduce their complexity since they are often based on a reduced number of genetic, pharmacological or environmental alterations that do not fully recapitulate the biological scenario of the pathology.

So, there is an unmet medical need to develop animal models that recapitulate the complexity of mental disease. Using bipolar disorder as a proof of concept, the inventors of the present invention have developed a method to generate a humanized animal model derived from cells of patients suffering from a mental disease that recapitulates the patient's symptomatology and treatment response.

### DESCRIPTION OF THE INVENTION

### Brief description of the invention

The present invention refers to an animal model of a mental disorder. As a proof of concept, the inventors have generated a humanized murine model of bipolar disorder that maintains the characteristics of the donor patient in terms of disease symptomatology and treatment response. This animal model has been achieved through the implantation of neural precursors derived from the olfactory neuroepithelium (hONE: human Olfactory Neuroephitelium) from patients with bipolar disorder into the brain of adult rodents. Thus, the inventors have incorporated the genetic and epigenetic deficits of the patient into a healthy mammal, reproducing in the latter the conditions of the donor patient. The present invention comprises the following phases:

### Phase 1: Culture of neural precursors of the human olfactory neuroepithelium

The olfactory neuroepithelium presents neural precursors, immature and mature olfactory neurons that reflect the genetic and epigenetic profile of an individual as well as of the pathology they suffer from (Benitez-king et al., 2016; Borgmann-winter et al., 2015). Unlike induced pluripotency stem cells, olfactory neuroepithelial neural progenitors do not require reprogramming to increase their differentiation potential, allowing them to retain the patient's own genetic and epigenetic information. Under physiological conditions in humans, they are able to differentiate into olfactory sensory neurons and glial cells provided a suitable molecular environment (Eisch et al., 2012). These cells have the advantage that they can be obtained by minimally invasive methods, without the need for surgery, by nasal brushing. This neuroepithelium sample includes a heterogeneous population of cells among which neural precursors are the ones with the highest proliferation rate. Thus, neural precursors constitute the predominant population once an olfactory neuroepithelium culture is established. Experimental trials are performed in subcultures 6-8, passages at which the culture is homogeneous and stable (Benítez-King et al., 2011).

The inventors of the present invention obtained samples of the olfactory neuroepithelium from patients with type I bipolar disorder (according to the Diagnostic and Statistical Manual of Mental Disorders, or DSM5, diagnostic criteria) as well as from control subjects. Patients with type I bipolar disorder were subdivided into two subgroups, those who are good responders to lithium treatment and those who are not according to Alda score (Grof et al., 2002).

### Phase 2: Implantation of human olfactory neural progenitors in a rodent neurogenic niche

To generate an animal model of bipolar disorder, the inventors implanted olfactory neuroepithelium-derived progenitors in the area adjacent to the hippocampus of nude mice. Nude mice were selected because they lack a thymus and are therefore immunodeficient, thereby reducing the chances of implant rejection.

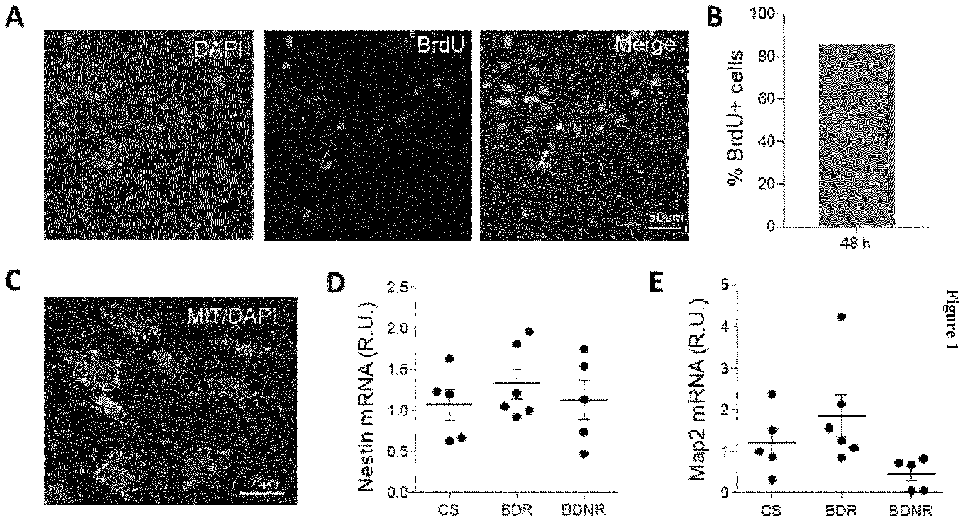

Cells that are obtained from the human olfactory neuroepithelium and then cultured under proliferating conditions are mostly Nestin+ precursors (Delgado-Sequera et al., 2021). A high percentage of these cells, around 85%, were found to proliferate during the 48 hours prior to their implantation in the hippocampus of nude mice and were detectable using an antibody against human mitochondria (anti-MIT) (Fig. 1A-C). At the time of implantation, cells derived from healthy controls (CS) and from patients with bipolar disorder - both responders (BDR) and non-responders (BDNR) to lithium treatment - displayed comparable levels of Nestin mRNA, indicating that the cell differentiation stage was similar across groups (**Fig**. **1D**). In addition, no significant differences in the expression of the neuronal marker Map2 were observed between groups (**Fig**. **1E**). These data indicate that the majority of implanted cells have proliferative capacity and are at a similar cell stage.

Athymic nude mice were anesthetized and olfactory neuroepithelial cells obtained from either healthy subjects or patients were implanted by stereotaxic surgery in an area close to the hippocampus. The implantation was performed in this area because previous studies have shown that the cells migrate from here to the mouse dentate gyrus, an area where endogenous neurogenesis occurs (Pilz et al., 2018). Thus, implantation of neural precursors in this area makes it possible to explore the effect of the integration and subsequent differentiation of human cells in a rodent neurogenic niche.

### Phase 3: Validation of the animal model

Initially, we studied whether the implanted cells were able to migrate to the hippocampus. hONE cells were detected in the hippocampus at 24 and 4 weeks after implantation (**Fig**. **2-3**) and in the subventricular zone at 4 weeks (**Fig**. **3**).

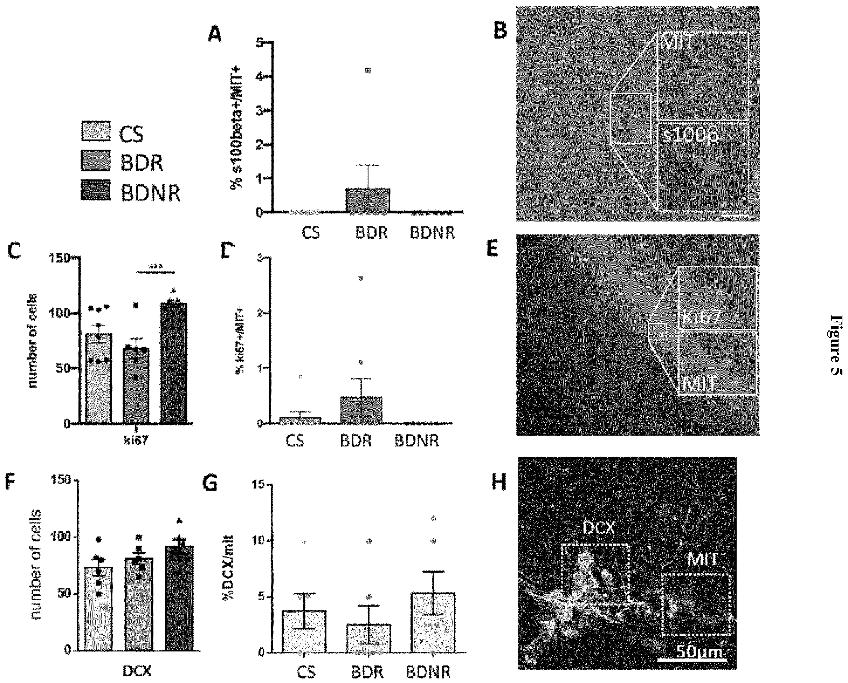

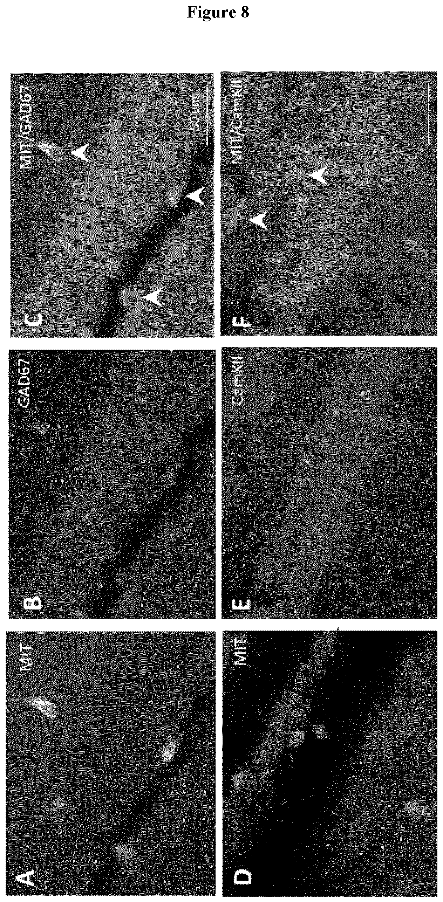

The same approach was carried out in animals injected with cells from healthy controls (CS), patients with bipolar disorder, lithium responders (BDR) and non-responders (BDNR). No major differences were detected in the total number of MIT+ cells at 4 weeks post implantation across the different experimental groups (**Fig**. **4A**). Further analysis of the distribution of these cells in the hippocampus revealed that the number of MIT+ cells in CA1 was higher than in CA3. Within CA1, a significantly higher number of cells was detected in BDNR compared to their BDR and CS counterparts (**Fig**. **4B**). Regarding the phenotype of hONE MIT+ cells labeled with ki67 (proliferation marker), S100β (glial marker) and doblecortin (DCX, immature neuron marker), their percentage of expression was very low and no significant differences were observed across experimental groups (**Fig. 5**). The expression of glial markers such as GFAP and Iba1 in the hippocampus was comparable to that detected in other areas, such as the piriform cortex, indicating that there were no differences in glial reactivity at 4 weeks after implantation across groups (**Fig**. **6**). Further studies showed that a high percentage of hONE cells present at the hippocampus expressed the NeuN marker, which is indicative of mature neurons, and that no significant differences in the abundance of NeuN+ cells was detected across groups. Implanted cells also presented staining for GABAergic and glutamatergic neuron markers (**Fig. 8**). In addition, the area occupied by MIT+ staining was larger in the bipolar groups with respect to the control (**Fig**. **7E**) and the MIT fluorescence intensity - normalized to the MIT+ area - was significantly higher in the BDNR group (**Fig**. **7F**). These results point to a larger size of BDR and BDNR cells compared to control cells, and to a higher intensity in the expression of mitochondrial markers in the BDNR group.

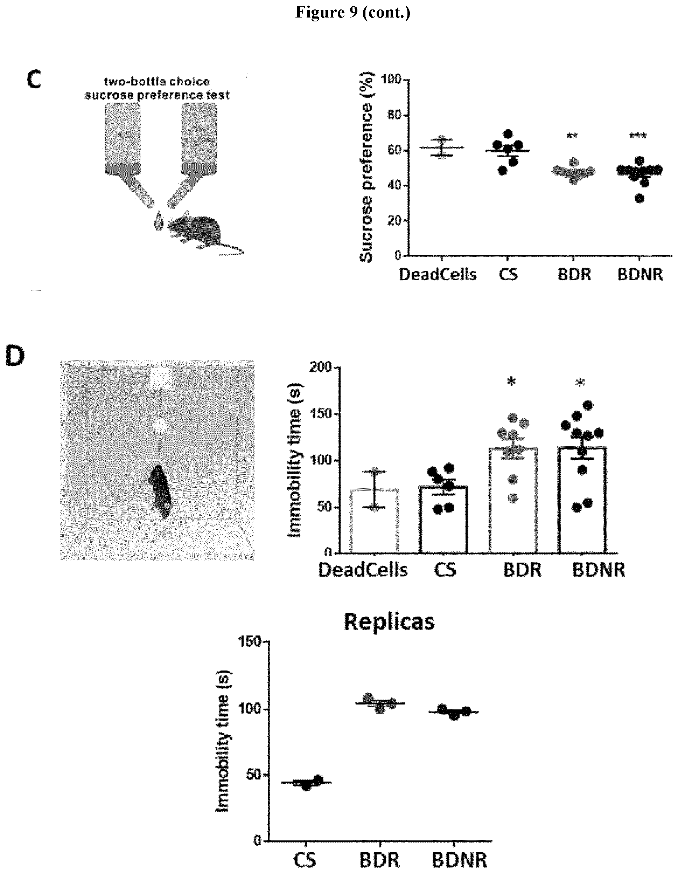

The inventors them aimed to characterize the behavioral phenotype developed by nude animals transplanted with human cells. Their studies show that animals transplanted with cells from patients with bipolar disorder showed motor hyperactivity (both during the day and during the night) (**Fig**. **9B**) and a depressive-like behavior. This depressive-like behavior was detected using anhedonic-like behavior (sucrose preference test) and behavioral hopelessness (tail suspension tests) tests, where replicates of the same sample of hONE showed high reproducibility (Fig. 9C-D). However, no cognitive deficit was detected in the new object recognition test (**Fig**. **9E**)**.** This behavioral profile demonstrates the apparent validity of the animal model of bipolar disorder as it mimics certain characteristics of the disorder symptomatology. No phenotypic differences were detected between animals implanted with cells from patients with type I bipolar disorder with good or bad response to lithium. As expected, treatment with the psychostimulant methylphenidate significantly increased motor activity in animals implanted with cells from non-responders and responders to lithium (**Fig**. **9B**). This experiment serves as a negative control and demonstrates the specificity of lithium treatment. The same procedure was performed by implanting the hONE cells at the level of the striatum and no phenotypic changes were found across bipolar groups with respect to the controls (**Fig**. **10**), demonstrating the specificity of the site of implantation.

To evaluate the predictive validity of the model, the effect of pharmacological treatment with lithium (reference drug in the treatment of bipolar disorder) was evaluated. The data show that lithium treatment specifically reversed motor hyperactivity and depressive-like behavior only in animals implanted with cells from good lithium responders, while it did not significantly modify this phenotype in mice implanted with cells from poor lithium responders (**Fig**. **11**). These data demonstrate that this method resulted in the generation of an animal model that reproduces the characteristics of the donor in terms treatment response.

As for the side effects generated by lithium treatment, these were more severe in the case of mice implanted with cells derived from non-responders, since a significant increase in plasma urea and alanine aminotransferase (ALT) - which are indicative of increased renal and hepatic dysfunction, respectively - was observed in these mice compared to mice implanted with cells derived from responder patients (**Fig**. **12**).

So, the first embodiment of the present invention refers to a method for generating a non-human animal model of a mental disorder comprising implanting human olfactory neural progenitors into a neurogenic niche (preferably hippocampus) of a non-human recipient animal.

In a preferred embodiment, the mental disorder is selected from: bipolar disorder, schizophrenia, psychotic disorders, major depressive disorder, autism and/or addictive disorders.

In a preferred embodiment, the mental disorder is bipolar disorder.

In a preferred embodiment, the method comprises the following steps:
a. culturing human olfactory neural progenitors,
b. implanting human olfactory neural progenitors into a neurogenic niche of a non-human recipient animal.

In a preferred embodiment, the recipient animal is a mammal, preferably a rodent or primate, more preferably a rat or a mouse.

In a preferred embodiment, the recipient animal is an immunodeficient or immunosuppressed mouse, preferably an immunodeficient BALB/c nude mouse.

The second embodiment of the invention refers to a non-human animal model obtained or obtainable through any of the methods described above.

The third embodiment of the invention refers a non-human animal model characterized in that it comprises human olfactory neural progenitors, or differentiated cells selected from glia cells and/or neurons derived from said neural precursors, in the brain.

In a preferred embodiment, the non-human animal model is characterized in that it displays a mental disorder phenotype.

In a preferred embodiment, the non-human animal model is characterized in that it displays a phenotype of bipolar disorder, schizophrenia, psychotic disorders, major depressive disorder, autism and/or addictive disorders.

The fourth embodiment of the invention refers to a method for screening candidate compounds useful in the treatment of a mental disorder, or for evaluating the efficacy of a preventive or therapeutic treatment against a mental disorder, comprising administering said candidate compound to any of the non-human animal models described above, wherein a positive response to treatment is indicative that the candidate compound could be used in the treatment of the mental disorder in humans or that the candidate compound is effective as a preventive or therapeutic treatment against the mental disorder in humans.

The fifth embodiment of the invention refers to the use of any of the non-human animal model described above to study the pathophysiology or behavior associated with a mental disorder.

The sixth embodiment of the invention refers to the use of human olfactory neural progenitors for the generation of a non-human animal model of a mental disorder.

In a preferred embodiment, the mental disorder is selected from bipolar disorder, schizophrenia, psychotic disorders, major depressive disorder, autism and/or addictive disorders.

In a preferred embodiment, the mental disorder is bipolar disorder.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.
- The term "mental disorder" refers to a behavioral or mental pattern that causes significant distress or impairment of personal functioning. For the purpose of this invention, the term "mental disorder" refers to those disorders reviewed in the Diagnostic and Statistical Manual of Mental Disorders (DSM-5) published by the American Psychiatric Association, which is considered one of the best-established systems for the classification of mental disorders. These mental disorders include: attention-deficit/hyperactivity disorder (ADHD), autism spectrum disorder, conduct disorder, disruptive mood dysregulation disorder, eating disorders, gender dysphoria, intellectual disability, internet gaming disorder, major depressive disorder and the bereavement exclusion, mild neurocognitive disorder, obsessive-compulsive and related disorders, paraphilic disorders, personality disorder, posttraumatic stress disorder, schizophrenia, sleep-wake disorders, specific learning disorder, social communication disorder, somatic symptom disorder and substance-related and addictive disorders.
- The term "human olfactory neural progenitors" refers to olfactory neuroepithelial cells characterized in that they are able to differentiate into olfactory sensory neurons and glial cells under physiological conditions. These cells have the major advantage that they can be obtained by minimally invasive methods, without the need for surgery, by nasal brushing. This sample of neuroepithelium represents a heterogeneous population of cells, among which neural precursors display a higher proliferation rate. Therefore, neural precursors constitute the predominant population in established cultures derived from olfactory epithelium samples.

### Description of the figures

**Figure 1****. Characteristics of hONE cells before implantation in mice.** A) Immunofluorescence of cultured cells labeled with anti-BrdU and nuclei labeled with DAPI. B) Quantification of the percentage of BrdU+ cells. C) Immunofluorescence of cultured cells labeled with an anti-mitochondria antibody specific for human protein and nuclei labeled with DAPI. D-E) RT-qPCR mRNA quantification of Nestin and Map2 expression levels in the different experimental groups. Graph shows mean ± SEM; ANOVA, p>0.05. CS: control subjects; BDR: lithium responder; BDNR: lithium non-responder; R.U.: relative units.

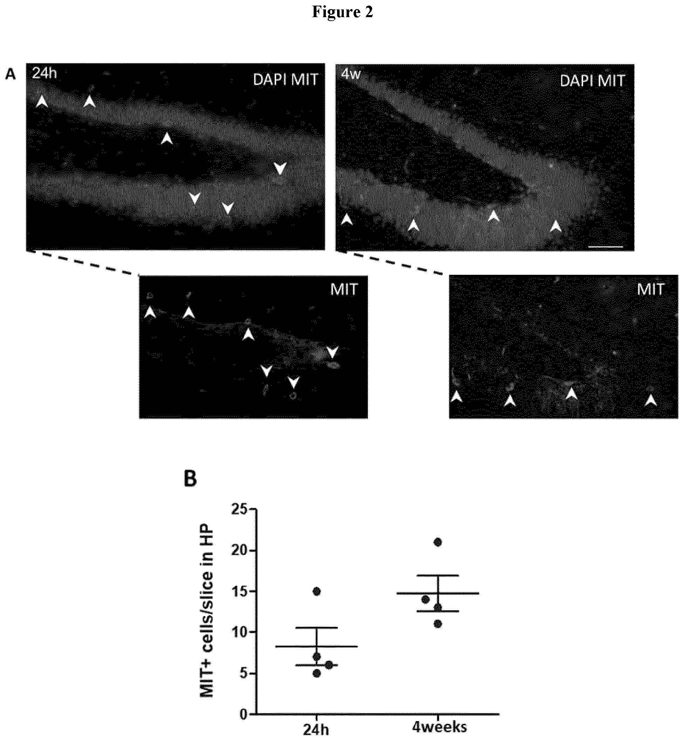

**Figure 2****. Anti-human mitochondria (MIT) staining in hONE cells inserted into the hippocampus of nude mice at 24 hours (24h) and 4 weeks (4w).** A) Representative fluorescence microscopy images of hONE cells inserted into the hippocampus of nude mice expressing MIT (arrow heads) and DAPI at 24h and 4 weeks. B) Quantification of the number of MIT+ cells in the hippocampus after 24h and 4 weeks. Graph shows mean ± SEM; Student's t, p>0.05. Scale bar: 50 µm.

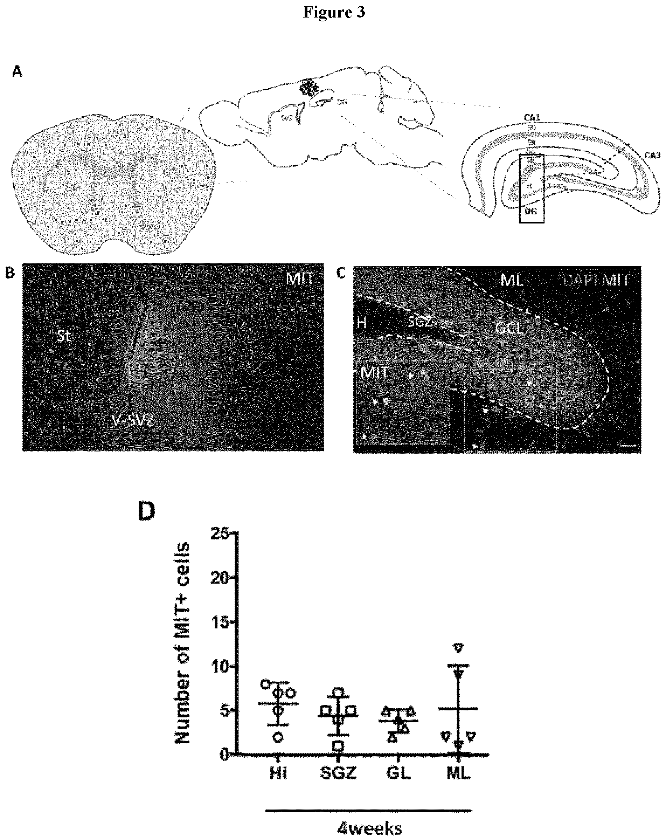

**Figure 3****. Distribution of human mitochondria (MIT)-positive cells in the brain of nude mice after 4 weeks.** A) Schematic of the localization of the hONE cell implantation site and adjacent areas susceptible to migration (endogenous neurogenic niches: subventricular zone (SVZ) of the lateral ventricles (V-SVZ) and subgranular zone of the dentate gyrus (DG-SGZ). B) Representative fluorescence microscopy image of hONE MIT+ cells in SVZ. C) Representative fluorescence microscopy image of hONE cells inserted in different areas of the hippocampal dentate gyrus expressing MIT (arrow heads) and DAPI. D) Graph showing the number of MIT+ cells at 4 weeks after implantation in the different regions of the dentate gyrus (DG); hilus (Hi), subganglionic layer (SGZ), granular layer (GL) and molecular layer (ML). Graph shows mean ± SEM; ANOVA, p>0.05. Scale bar: 20 µm.

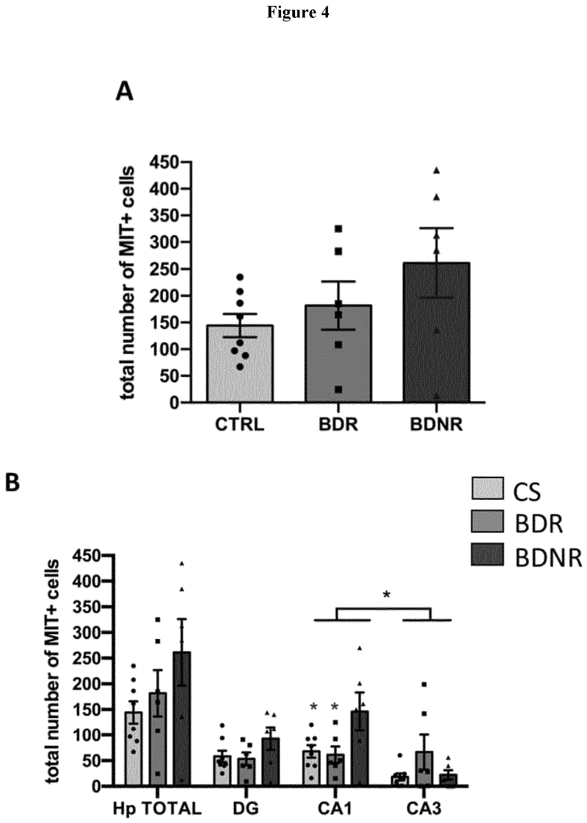

**Figure 4****. Total number and distribution of human mitochondrial (MIT)-positive cells from control subjects (CS), bipolar disorder patients, lithium responders (BDR) and lithium non-responders (BDNR) in the hippocampus of nude mice 4 weeks post-implantation.** A) Graph showing the total number of MIT+ cells in nude mice implanted with cells from CS, BDR and BDNR. B) Graph showing the number of MIT+ cells in nude mice implanted with cells from CS, BDR and BDNR in the dentate gyrus (DG), CA1 (Cornu Ammonis, 1) and CA3 (Cornu Ammonis, 3). Graphs show mean ± SEM; ANOVA, *p<0.05.
**Figure 5****. Phenotype of human mitochondrial (MIT)-positive cells from control subjects (CS), bipolar disorder patients, lithium responders (BDR) and lithium non-responders (BDNR) in hippocampus of nude mice 4 weeks post-implantation.** A) Percentage of MIT+ cells co-expressing S100β+ (immature glia marker) in hippocampus. B) Representative fluorescence microscopy image of a hONE cell inserted in the molecular layer of the hippocampal dentate gyrus expressing MIT and S100β. (C) Number of Ki67+ cells (proliferation marker) in the hippocampal dentate gyrus. D) Graph showing the percentage of MIT+ cells co-expressing ki67 in CTRL, BDR and BDNR. E) Representative fluorescence microscopy image of a hONE cell inserted in the molecular layer of the hippocampal dentate gyrus expressing MIT and Ki67. F) Graph showing the number of doublecortin (DCX)-positive neurons per slice. G) Graph showing the percentage of MIT+ cells co-expressing DCX. H) Representative confocal microscopy images showing hONE cells embedded in the hippocampal granular layer expressing MIT, DCX+ cells and DAPI. Graphs show mean ± SEM; ANOVA, ***p<0.001. Scale bar (B, E): 40 µm.

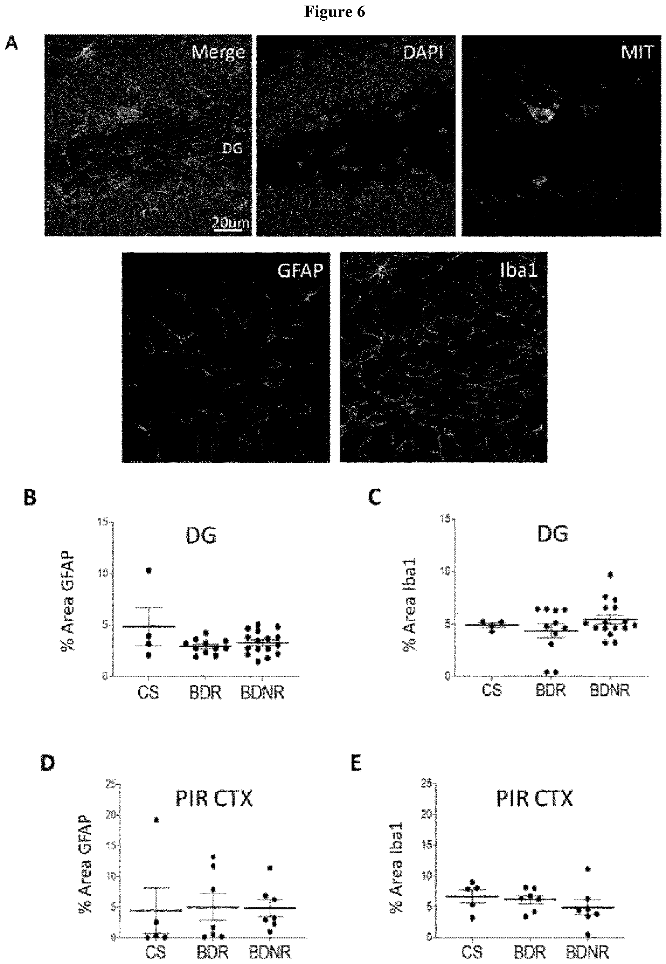

**Figure 6****. Immunofluorescence studies 4 weeks after hONE implantation.** A) Representative image of the dentate gyrus (DG) with MIT-labeled human cells, GFAP-labeled astrocytes and Iba1-labeled microglia. B) Quantification of the percentage of area occupied by GFAP in the DG. C) Quantification of the percentage of area occupied by IBA1 in the DG. D) Quantification of the percentage of area occupied by GFAP in the pyriform cortex (PIR CTX). E) Quantification of the percentage of area occupied by IBA1 in the piriform cortex. Graphs show mean ± SEM; ANOVA, p>0.05. CS: control subjects; BDR: lithium-responsive patient; BDNR: lithium non-responsive patient.

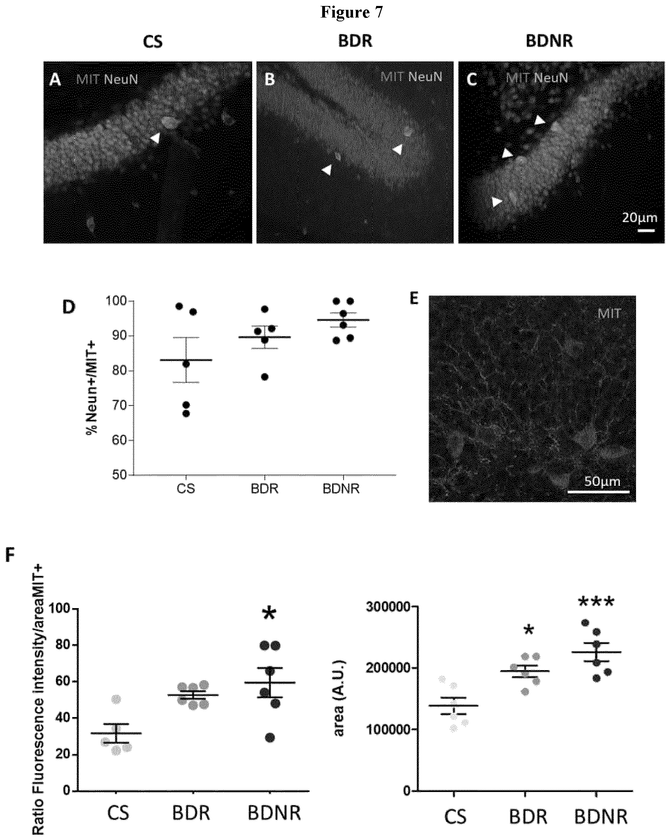

**Figure 7****. hONE MIT+ cells mainly express the mature neuron marker NeuN.** A-C) Mature neurons were labeled using NeuN antibody and hONE cells were labeled by anti-MIT. NeuN+/MIT+ co-expressing cells are marked by arrow heads. D) Quantification of the percentage of cells co-expressing the human MIT marker and the neuronal marker NeuN in CS, BDR and BDNR. E) Area of MIT+ cells. F) Quantification of the relationship between fluorescence intensity for the MIT marker and MIT+ cell area. Graphs show mean ± SEM; ANOVA, *p<0.05, ***p<0.001 vs CS. CS: control subject; BDR: lithium responder; BDNR: lithium non-responder. A.U.: arbitrary units.
**Figure 8****. Immunofluorescence studies 4 weeks after hONE implantation.** A-C) Representative image of the GABAergic neuron marker GAD67 and the human mitochondrial marker, MIT. D-F) Representative image of the glutamatergic neuron marker CAMKII and the human mitochondrial marker, MIT. Arrow heads indicate double positive cells.

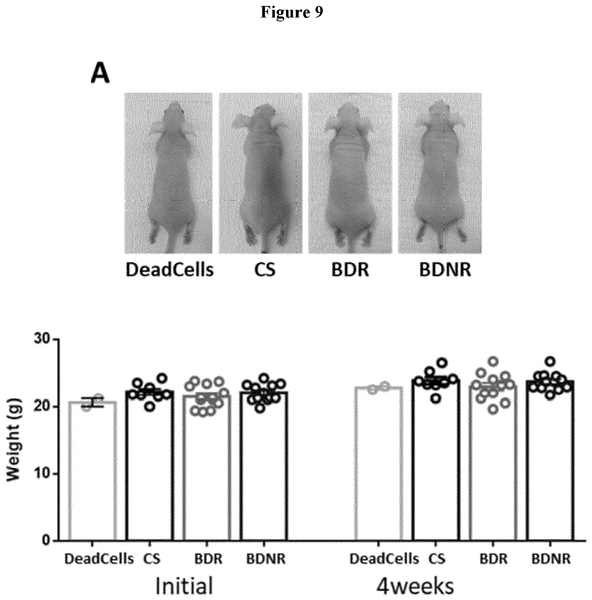

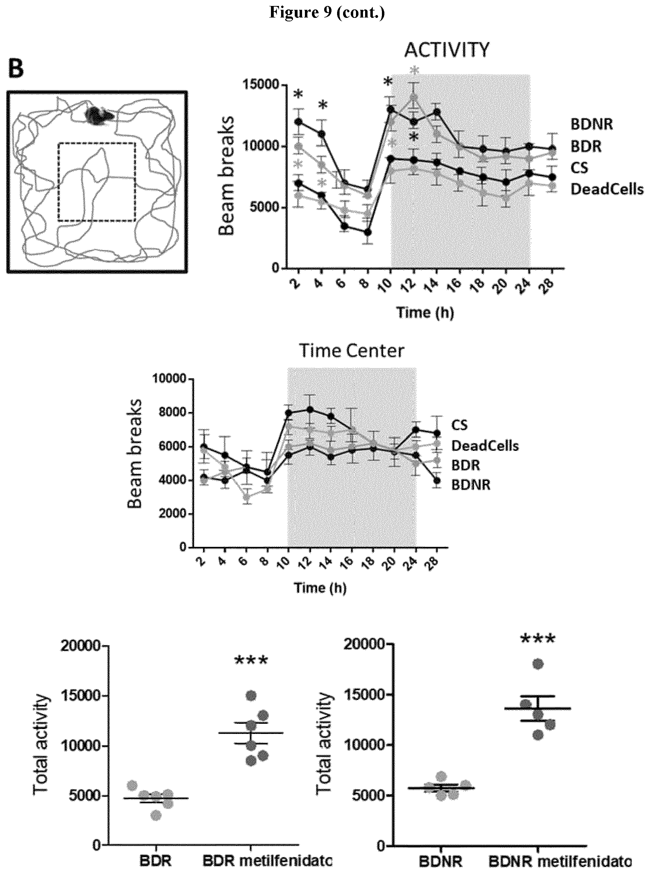

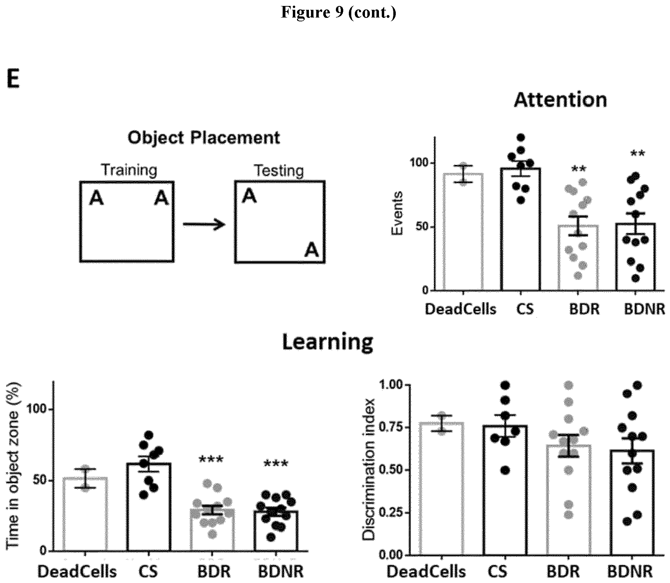

**Figure 9****. Behavioral characterization in nude animals 4 weeks after implantation of dead (as methodological control) or viable hONE cells from control subject (CS), bipolar responder (BDR) or non-responder (BDNR) patients in hippocampus.** A) Representative images of nude mice from the different experimental groups. Graph showing weight at implantation and after 4 weeks. B) Motor activity during 24 hours. Exploration of motor activity after methylphenidate administration in BDR and BDNR (90 min). C-D) Evaluation of depressive behavior: C) Sucrose preference test. D) Tail suspension test. Study of phenotype reproducibility: replicates of the same sample of hONE cells. E) Cognitive evaluation by means of the new object recognition test. Data are represented as mean ± SEM. ANOVA, *p<0.05, **p<0.01,***p<0.001 vs control. A.U.: arbitrary units.

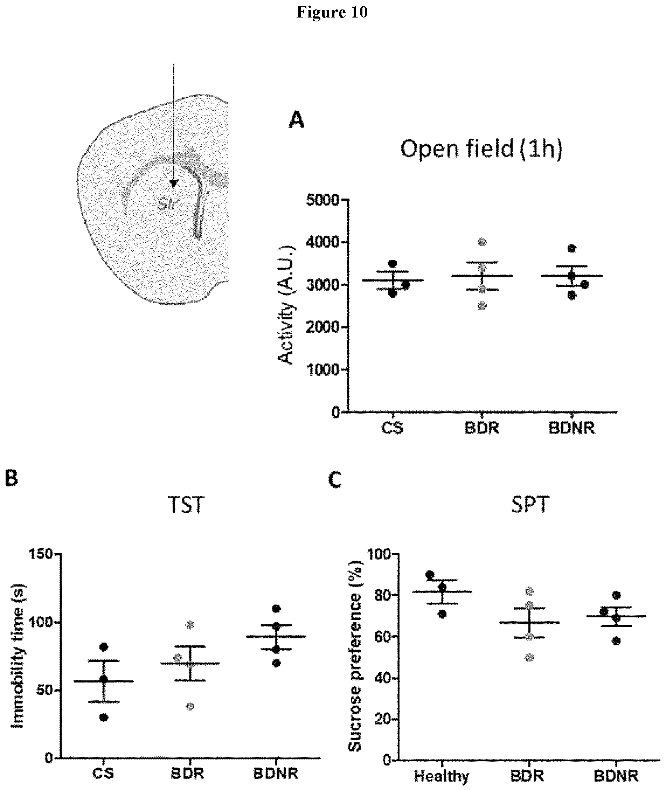

**Figure 10****. Behavioral characterization in nude animals 4 weeks after implantation of dead (as methodological control) or viable hONE cells from control subjects (CS), patients with bipolar responder disorder (BDR) or non-responder (BDNR) in the striatum.** A) Motor activity for 1 hour. B-C) Assessment of depressive-like behavior: B) Tail suspension test. C) Sucrose preference test. Data are represented as mean ± SEM. ANOVA, p>0.05. A.U.: arbitrary units.

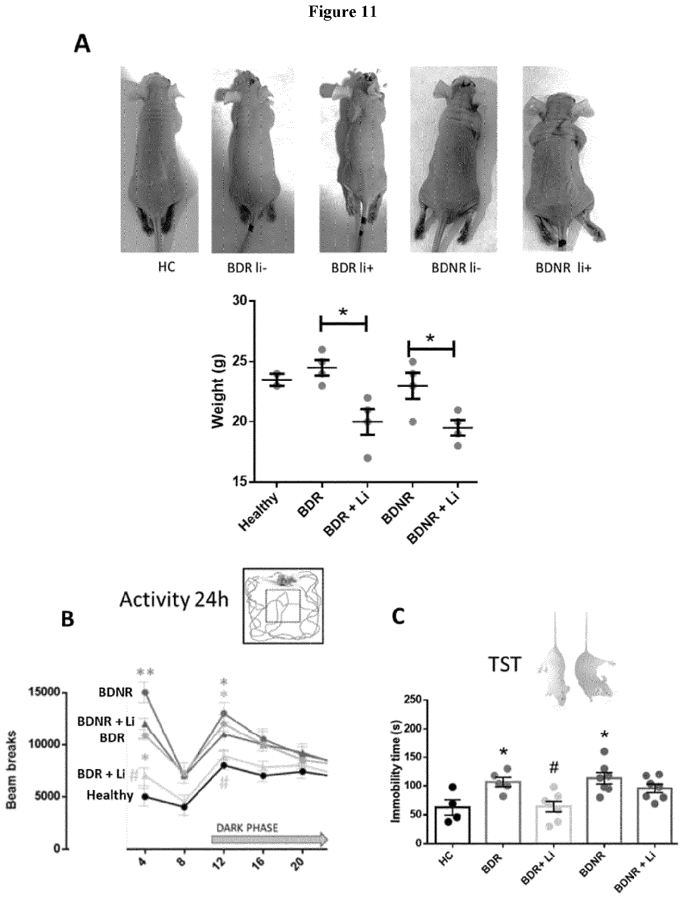

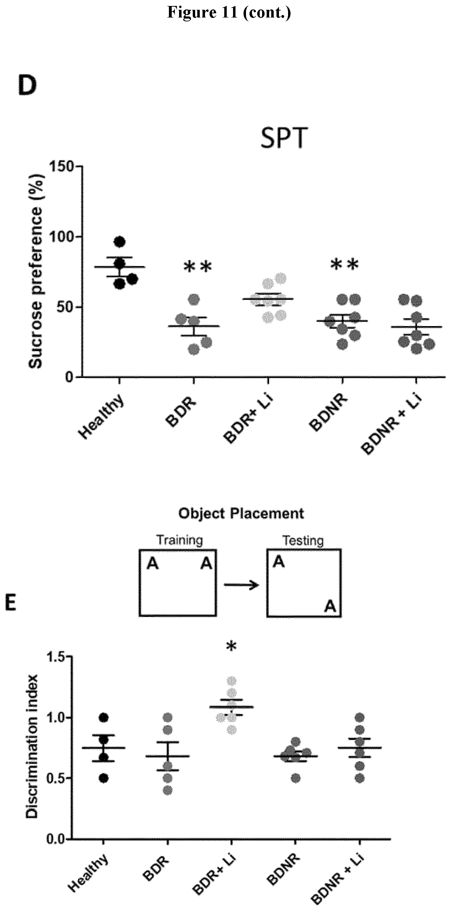

**Figure 11****. Pharmacological study with baseline treatment for bipolar disorder in nude mice with hONE cells from control subjects (CS), patients with bipolar disorder responder (BDR) or non-responder (BDNR) in the hippocampus. The animals were treated with lithium for 3 weeks (lithium feed 0.2%).** A) Effect of treatment on weight. B) Behavioral effect of lithium on spontaneous motor activity during 24h. C) Behavioral effect of lithium on the tail suspension test (TST, depressive behavior). D) Behavioral effect of lithium on the sucrose preference test (SPT). E) Behavioral effect of lithium on a cognitive test of novel object recognition. Data represent mean ± SEM, ANOVA, *p<0.05, **p<0.01 vs CS; # vs untreated group. A.U.: arbitrary units.

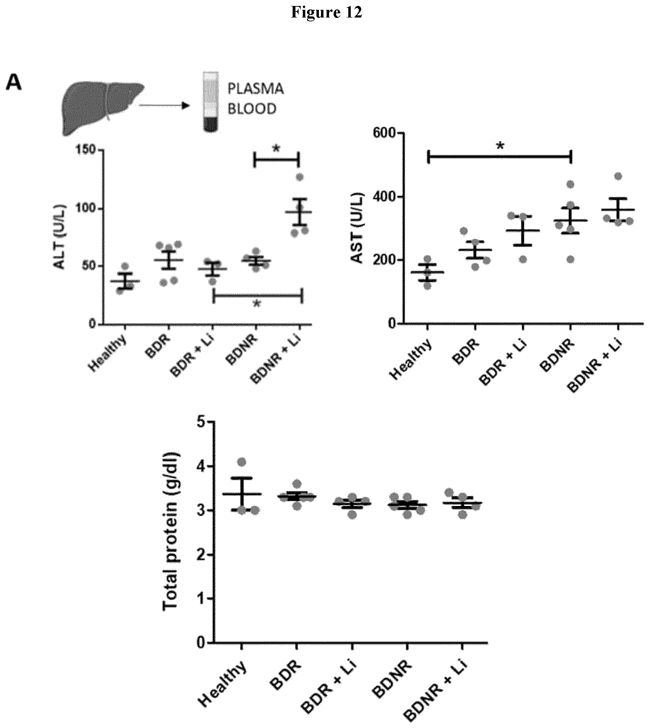

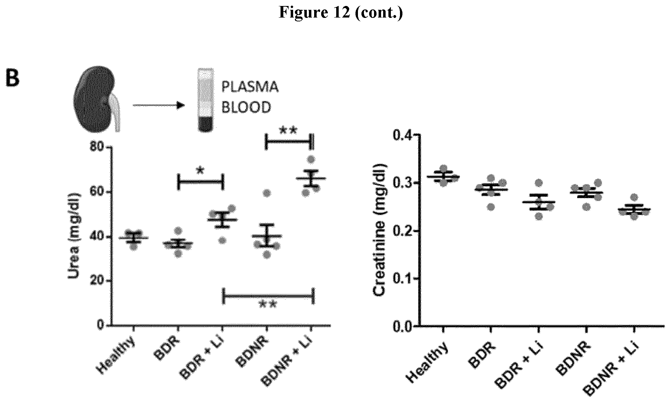

**Figure 12****. Biochemical analysis of plasma of nude mice after lithium treatment.** A) Levels of ALT (alanine aminotransferase), aspartate aminotransferase (AST) and total protein. B) Urea and creatinine levels. Data represent mean ± SEM, ANOVA, *p<0.05, **p<0.01 vs control; # vs untreated group.

### Detailed description of the invention

The following specific Examples provided in this patent serve to illustrate the nature of the present invention. These Examples are included for illustrative purposes only and are not to be understood as limitations to the invention. Thus, the Examples described below illustrate the invention without limiting its scope.

### Example 1: Culture of neural precursors of human olfactory neuroepithelium

Neural precursors of the olfactory neuroepithelium were obtained from control subjects and from patients with type I bipolar disorder (according to diagnostic criteria of the Diagnostic and Statistical Manual of Mental Disorders, DSM-5). The response to Lithium treatment was measured using the scale called "Retrospective Criteria of Long-Term Treatment Response in Research Subjects with Bipolar Disorder", designed by M. Alda (Grof et al., 2002; Schulze et al., 2010; Manchia et al., 2013). This scale quantifies the degree of clinical improvement after treatment with this drug using retrospective information. It consists of 11 items that are divided into two criteria; criterion A is expressed as a composite score of change in frequency and severity of affective symptoms. This criterion is weighted by criterion B which measures the degree of attribution of clinical improvement to treatment.

*Inclusion criteria for the group of patients classified as responders or non-responders to lithium treatment:*
- Age between 18 and 70 years.
- Having a diagnosis of type I Bipolar Disorder according to DSM-5 criteria evaluated from the data obtained in the clinical history of the patients and after the completion of the semi-structured interview SCID-5-CV (see below).
- Being in treatment with Lithium in monotherapy for at least 1 year (prior to the interview).
- For the group "good responders to lithium treatment", a total score equal to or greater than 7 on the scale "Retrospective Criteria of Long-Term Treatment Response in Research Subjects with Bipolar Disorder" will be considered as such. For the group "poor responders to Lithium treatment", a total score equal to or less than 3 on the same scale will be considered as such.
- Speaking Spanish correctly.

*Exclusion criteria for the group of patients classified as responders or non-responders to lithium treatment:*
Presence of organic diseases of the central nervous system (CNS) or history of cranioencephalic trauma with loss of consciousness. Mental retardation. Pervasive developmental disorders. Pregnancy and lactation.

### For the control group:

Healthy subjects, without psychiatric diagnosis, with the same exclusion criteria as the patient group and the same sociodemographic profile (sex, age, socioeconomic level).

Neuroepithelial cells were extracted from the nasal cavity using a procedure consisting of brushing the inferior and middle turbinate in a circular motion after localization of the area using a fiberscope. These extracted cells were cultured at 37 °C and 5% CO2 in Dulbecco's Modified Eagle Medium/Ham F-12 (DMEM/F12) with 10% fetal bovine serum (FBS), 2% GlutaMAX 100X and 0.2% primocin (Invivogen) (Delgado-Sequera et al., 2021; Benitez-King et al., 2011). Once 80% confluence is reached, the culture is dissociated with 0.25% trypsin and subcultured.

Among the different cell types extracted, neural precursors have the highest proliferation rate and will be the predominant population once the culture is established. After several subcultures, homogeneity is obtained in the predominant cell type which, as mentioned, are Nestin+ neural precursors (Delgado-Sequera et al., 2021). In experimental trials in which cells were injected into the hippocampus of nude mice, cells were used in subculture 6-8, passages at which the culture is expected to be homogeneous and stable (Benítez-King et al., 2011).

To verify that the cells prior to implantation maintained their proliferative capacity, a BrdU incorporation study was performed. For this purpose, cells were incubated at 37 °C and 5% CO2 with BrdU present in the culture medium. After 48 h, cells were harvested for implantation in the hipocampus and others were fixed for immunofluorescence with 4% paraformaldehyde (PFA) in PBS for 20 min. After PBS washes, cells were incubated for 1 hour at room temperature in a blocking solution composed of 0.25% Triton-X-100 and 10% FBS in PBS. The cells were then incubated overnight at 4 °C with a mouse anti-BrdU antibody (DAKO; M0744). After washes with PBS, cells were incubated with the fluorescently labeled secondary antibody for 1 h at room temperature. Nuclei were stained with 4',6'-diamidino-2-phenylindole dihydrochloride (DAPI). After quantification of the number of BrdU+ cells with respect to the total number of cells labeled with DAPI, we observed that 85% of cells had incorporated BrdU into their DNA, i.e., they had divided during the previous 48 hours, indicating that they maintained their proliferative capacity (Fig. 1A-B; quantification performed in triplicate). In addition, all cultured cells were found to be labeled with anti-human mitochondrial antibody (1:500,Millipore, USA), an antibody that was subsequently used to label these cells specifically in the mouse hippocampus (**Fig**. **1C**).

To analyze the stage of cell differentiation in the different experimental populations, an mRNA expression study was performed for the genes Nestin (marker of neural proliferation) and Map2 (marker of differentiated neurons). For this purpose, quantitative RT-PCR was performed with oligonucleotides specific for Nestin (SEQ ID NO: 1 Nestin_forward 5'-GGGAAGAGAGAGGTGATGGAACCA-3'; SEQ ID NO: 2 Nestin reverse 5'-AAGCCCTGAACCCTCTCTTTGC-3'), for Map2 (SEQ ID NO: 3 Map2_forward 5'-TTGGTGGTGCCGAGTGAGAAGAAGA-3'; SEQ ID NO: 4 Map2_reverse 5'-GTCTGGGCAGTGGTTGGTTGGTTAA-3') and RPL0 (SEQ ID NO: 5 RPL0_forward 5'-CAGATTGGCTACCCAACTG-3'; SEQ ID NO: 6 RPL0_reverse 5'-GGGAAGGTGTAATCCGTCT-3') which was used as an endogenous control for normalization. SYBRgreen (Bio-Rad), and 100ng of cDNA per sample were used for PCR. The analysis was performed following the ddCT method (Livak et al., 2001). The result obtained indicated that cells derived from healthy controls and patients have similar level of Nestin expression at the time of injection (Fig. 1D; ANOVA, p>0.05; CS=5; BDR=6; BDNR=5), as well as Map2 expression (Fig. 1E; ANOVA, p>0.05; CS=5; BDR=6; BDNR=5).

### Example 2: Implantation of olfactory neuroepithelial cells in the hippocampus of nude mice

Immunodeficient BALB/c nude mice (CAnN.Cg-Foxn1nu/Crl) developed through crosses and backcrosses between BALB/cABom-nu and BALB/cAnNCrj-nu at Charles River Laboratories were used. The production colony was generated from pedigreed pregnant females of CAnN.Cg-Foxn1nu/Crll. This mouse was inbred, and genetic monitoring results confirmed that it was a BALB/c nude. The animal lacks a thymus, is unable to produce T cells and is therefore immunodeficient.

Athymic nude mice (8 weeks old, Charles River Laboratories) were anesthetized with isofluorane and by stereotaxic surgery olfactory neuroepithelium cells from healthy subjects or patients are implanted. These cells were injected using Hamilton syringe coupled to an infusion pump. The inventors performed the implantation of 250,000 cells/microliter at a flow rate of 1ul/min, 1microliter per hemisphere in the area adjacent to the hippocampus (coordinates with respect to the bregma: AP-2.3AP, L± 1.3, V-2.0 (Bueno et al., 2013) and with a delay of at least 2min, to avoid losses with syringe recoil. Pre-post injection the inventors made sure to clean with ethanol the administration syringe. Thus, the implantation of neural precursors in this area makes it possible to explore the effect of integration and subsequent differentiation of human cells in the rodent neurogenic niche, which was analyzed after 4 weeks.

Initially, to check the survival and migration of hONE cells from young healthy controls (<30 years) implanted in the hippocampus, the inventors performed immunohistochemical analysis 24 hours and 4 weeks after surgery (**Fig**. **2**). After this time, athymic nude mice were sacrificed by intracardiac perfusion with NaCl (0.9%), followed by 4% paraformaldehyde (PFA), after deep anesthesia with pentobarbital (1ml/kg). The brains of these animals were removed, fixed (2h, PFA 4%) and cryoprotected using a 30% sucrose solution. Brains were then cut into 40 µm thick coronal sections using the freezing microtome (HM 450, Thermo Scientific, UK) and collected serially in microtubes with cryoprotective solution (40% phosphate buffer (PB), 30% ethylene glycol, 30% sucrose). Immunofluorescence was then performed to detect the presence of hONE cells. First, the tissue was washed with PBS (3 washes of 5 minutes under agitation). Next, antigen unmasking was performed by incubating the tissue with citrate buffer (10m M sodium citrate, 0.05% Tween 20, pH 6.0) at 95-100° C, for 15 minutes under agitation, followed by 3 washes of 5 minutes under agitation with PBS. For blocking nonspecific binding, the tissue was incubated in 10% NDS in 0.2% Triton 0.2% PBS for 1 hour under agitation. After blocking the sections were incubated in the primary antibody, mouse-produced anti-human mitochondrial antibody (MIT) (1:500, Millipore, USA), in 5% NDS in 0.2% PBS triton for 1 night at 4°C and agitation.

On the second day, the tissue was washed with PBS (3 washes of 5 minutes with agitation) and then the sections were incubated with an appropriate biotinylated secondary antibody (biotinylated donkey anti mouse, 1:400, Jackson Immunoresearch, UK) in 5% NDS in 0.2% triton PBS for 1 hour with agitation. After this time and after 3 washes of 5 minutes with PBS, the sample was incubated with streptavidin conjugated with Alexa Fluor 555 ^{®} (1:1000, Invitrogen, USA), 1 hour under shaking and dark conditions. Three 5-minute washes with PBS were performed and then the sample was incubated with Sudan black (Sigma) (0.1% in 70% ethanol) to reduce autofluorescence, subsequently the tissue was incubated with DAPI 1:5000 (Sigma Aldrich, USA) for 5 minutes under shaking and dark conditions, the sample was washed twice for 5 minutes with PBS and once for 5 minutes with PB and the sections were incubated for 24 hours, static at 4°C. Sections were mounted on slides (StarFrost, Waldemar Knittel, Germany) and covered with appropriate mounting medium (Electron Microscopy Sciences EMS, USA). Sections processed for immunofluorescence were observed with the fluorescence microscope equipped with a digital camera (Olympus BX60, Olympus Opticsl Co., Japan) using the 40x objective. Photographs were taken in coronal sections in which the presence of cells labelled with anti-human mitochondrial antibody (MIT+) was observed and used to count total h-ONE MIT+ cells and study their distribution in the following hippocampal zones: molecular layer (ML), granular cell layer (GL), subgranular layer (SGZ) and hilus (Hi) (**Fig**. **3**). MIT+ cells were also detected in the subventricular zone. No tissue damage due to implantation, uncontrolled cell growth, ectopic cell growth or abnormal cell masses were observed in any of the transplanted animals (**Fig**. **3**). All these results indicate that hONE cells implanted in the adjacent hippocampal zone survive and are able to migrate.

### Example 3: Animal model validity tests

The same approach was then carried out in mice implanted with cells from control subjects (CS, n=8 mice), lithium-responsive (BDR, n=6 mice) and lithium-unresponsive (BDNR, n=6 mice). Four weeks after the injection of hONE cells into the hippocampus of athymic nude mice, mice were sacrificed, and their brains processed and analysed by immunofluorescence. Briefly, mice were perfused with saline at sacrifice. Brains were removed and fixed with 4% PFA for 48 hours, and then kept at 4°C in 30% sucrose. Coronal brain sections from the region containing the hippocampus were cut with a microtome (40 µm cutting thickness) and collected in six series. The immunohistochemistry protocol has been previously described (Bravo et al., 2022; Llorca-Torralba et al., 2019). Immunofluorescence was performed to characterize cells implanted in the hippocampus of mice, using the following markers: human cell marker (MIT), proliferative cell markers (Ki67), glial cells (S100 β), immature (DCX) or mature neurons (NeuN).

Using the anti-MIT antibody, the inventors observed that there were no differences in the number of total hONE (MIT+) cells between the three experimental conditions (CS, BDR and BDNR) after 4 weeks of implantation (**Fig**. **4A**). They also observed that the inserted human cells were distributed in all layers and regions of the hippocampus, although there was a higher number of MIT+ cells in CA1 compared to CA3. The number of MIT+ cells within CA1 was higher in mice injected with BDNR cells compared to their CS and BDR counterparts. (**Fig**. **4B**). In addition, no signs of tissue damage from implantation, uncontrolled cell growth, ectopic or abnormal cell masses were observed in any of the transplanted animals.

At 4 weeks after implantation, immunofluorescence studies showed low co-expression of MIT with Ki67 and S100beta in the CS, BDR, and BDNR groups, indicating that in all groups there were few proliferating hONE cells or cells that could be considered glial cells (**Fig**. **5**). However, the inventors did observe a significant increase in Ki67+/MIT- cells, in mice with BDNR cells, indicating that in these mice there was increased proliferation in mouse cells (**Fig**. **5C**). As for DCX+ immature neurons, there were also no differences between the CS, BDR and BDNR groups in terms of co-expression with MIT (Fig. 5F-H). In addition, at 4 weeks after implantation, neither microglia (IBA1) nor astrocyte (GFAP) activation was observed near the grafted cells that integrated into the hippocampus (**Fig 6A**). No significant difference in GFAP or IBA1 labeling was observed in the CS, BDR, and BDNR experimental groups after quantification of the DG and the piriform cortex as the basal labeling reference area (**Fig**. **6A-****E**). After 4 weeks of implantation, the inventors found that a high percentage of MIT+ cells express the mature neuron marker NeuN, indicating that the majority of implanted hONE cells differentiate to neurons (**Fig**. **7**). No significant differences in neuronal differentiation were observed between experimental groups, with a percentage of MIT+/NeuN+ co-expression of 81% in CS, 90% in BDR and 93% in BDNR (**Fig**. **7D**; CS=5; R=5; NR=6). Quantification of the area occupied by the MIT+ antibody showed greater area of occupancy in the BDR (p<0.05) and BDNR (p<0.001, **Fig**. **7E**) groups. In addition, MIT+ cells in the BDNR group showed a significant increase in the intensity of mitochondrial labeling (**Fig**. **7F**). GABAergic (GAD67+) and glutamatergic (CAMKII+) cells were observed 4 weeks after cell implantation, as well as co-expression of MIT with these cell markers (Fig. 8 A-F).

Next, a characterization of the behavioral phenotype developed by the nude animals after 4 weeks of CS, BDR and BDNR cell implantation was performed. There were no differences in the weight of the animals in the different experimental groups (**Fig**. **9A**)**.** Widely described tests were used to evaluate certain animal behaviors: 24h motor activity using open field test; anhedonia using sucrose preference test; behavioral despair through the tail suspension test and learning ability using a new object recognition test. Considering the symptoms of bipolar disorder in humans, the main behavioral phenotype related to BD is motor hyperactivity (correlate of mania) and depressive behavior (correlate of depression). Importantly, mice implanted with cells derived from BD patients showed motor hyperactivity (**Fig**. **9B**), anhedonia (sucrose preference test) (p<0.01 BDR vs CS; p<0.001 BDNR vs CS; **Fig**. **9C**) and behavioral despair (tail suspension test) (p<0.05 BDR and BDNR vs CS; **Fig**. **9D**). However, no significant differences were detected in the novel object recognition test (**Fig**. **9E**). Importantly, no differences were found between BDR or BDNR mice. As a control, dead ONE cells were also implanted in the hippocampus and no behavioral differences were observed compared to CS (**Fig**. **9**). As expected, methylphenidate administration produced an increase in motor activity in both BDR and BDNR with respect to the same untreated group (p<0.001; **Fig**. **9B**). In addition, several mice were engrafted with cells from the same donor, showing high reproducibility in behavior among them (CS=4; BDR=4; BDNR=3; **Fig 9D**). The same implantation procedure was performed at the level of the striatum, with no behavioral phenotype developing (**Fig**. **10**).

After performing this model behavioral characterization, the animals were chronically treated with lithium (3 weeks) mimicking the clinical situation. Lithium treatment significantly reduced the weight of the animals in both the BDR and BDNR groups (p<0.05, **Fig. 11**). Surprisingly, behavioral studies showed a normalization of motor activity and depressive phenotype only in the BDR group (Fig. 11 B-C). These results are consistent with the stratification of patients on the ALDA scale. In new object recognition, there was an improvement in the discrimination index, but only in the lithium-treated BDR group (p<0.05, **Fig. 11E**).

Biochemical analyses were performed on plasma samples from nude mice, where the renal profile (creatinine and urea levels) and liver profile (alanine aminotransferase (ALT), aspartate aminotransferase (AST) and total protein levels) were analyzed. These analyses showed that BDNR nude mice treated with lithium had a significant increase in urea and ALT, demonstrating that ONE cells implanted in the hippocampus have an effect at the peripheral level (**Fig**. **12**; CS=4; BDR=4; BDR+Li=4; BDNR=4; BDNR+Li=4). This result indicates that side effects related to lithium treatment (renal and hepatic dysfunction) are more pronounced in mice with BDNR patient cells. These results would demonstrate the predictive validity of this model and the possibility of using it to search for specific markers of treatment response.

### Applicability:

The present invention provides a method to generate animal models of a mental disease. As a proof of concept, it provides an animal model of bipolar disorder that recapitulates the specific characteristics and therapeutic response of the donor patient, thereby solving the lack of animal models for the study of bipolar disorder. This invention has applicability in other mental disorders with an etiopathogenic basis where there is a combination of genes and environment such as schizophrenia, psychotic disorders, major depressive disorder, autism, addictive disorders, among others.

### REFERENCES

Benitez-King, G., Valdés-Tovar, M., Trueta, C., Galvan-Arrieta, T., Argueta, J., Alarcon, S., ... & Solís-Chagoyán, H. (2016). The microtubular cytoskeleton of olfactory neurons derived from patients with schizophrenia or with bipolar disorder: Implications for biomarker characterization, neuronal physiology and pharmacological screening. Molecular and Cellular Neuroscience, 73, 84-95.

Bravo, L., Mariscal, P., Llorca-Torralba, M., López-Cepero, J. M., Nacher, J., & Berrocoso, E. (2022). Altered expression of vesicular glutamate transporter-2 and cleaved caspase-3 in the locus coeruleus of nerve-injured rats. Frontiers in Molecular Neuroscience, 15*.*

Bueno, C., Ramirez, C., Rodríguez-Lozano, F. J., Tabarés-Seisdedos, R., Rodenas, M., Moraleda, J. M., ... & Martinez, S. (2013). Human adult periodontal ligament-derived cells integrate and differentiate after implantation into the adult mammalian brain. Cell Transplantation, 22(11), 2017-2028.

Borgmann-Winter, K., Willard, S. L., Sinclair, D., Mirza, N., Turetsky, B., Berretta, S., & Hahn, C. G. (2015). Translational potential of olfactory mucosa for the study of neuropsychiatric illness. Translational psychiatry, 5(3), e527-e527.

Delgado-Sequera, A., Hidalgo-Figueroa, M., Barrera-Conde, M., Duran-Ruiz, M. C., Castro, C., Fernández-Avilés, C., ... & Berrocoso, E. (2021). Olfactory neuroepithelium cells from cannabis users display alterations to the cytoskeleton and to markers of adhesion, proliferation and apoptosis. Molecular Neurobiology, 58, 1695-1710.

Eisch, A. J., & Petrik, D. (2012). Depression and hippocampal neurogenesis: a road to remission?. Science, 338(6103), 72-75.

Grof, P., Duffy, A., Cavazzoni, P., Grof, E., Garnham, J., MacDougall, M., ... & Alda, M. (2002). Is response to prophylactic lithium a familial trait?. Journal of Clinical Psychiatry, 63(10), 942-947.

Livak, K. J., & Schmittgen, T. D. (2001). Analysis of relative gene expression data using real-time quantitative PCR and the 2- ΔΔCT method. methods, 25(4), 402-408.

Llorca-Torralba, M., Suárez-Pereira, I., Bravo, L., Camarena-Delgado, C., Garcia-Partida, J. A., Mico, J. A., & Berrocoso, E. (2019). Chemogenetic silencing of the locus coeruleus-basolateral amygdala pathway abolishes pain-induced anxiety and enhanced aversive learning in rats. Biological psychiatry, 85(12), 1021-1035.

Manchia, M., Adli, M., Akula, N., Ardau, R., Aubry, J. M., Backlund, L., ... & Alda, M. (2013). Assessment of response to lithium maintenance treatment in bipolar disorder: a Consortium on Lithium Genetics (ConLiGen) report. PloS one, 8(6), e65636.

Pilz, G. A., Bottes, S., Betizeau, M., Jörg, D. J., Carta, S., Simons, B. D., ... & Jessberger, S. (2018). Live imaging of neurogenesis in the adult mouse hippocampus. Science, 359(6376), 658-662.

Schulze, T. G., Alda, M., Adli, M., Akula, N., Ardau, R., Bui, E. T., ... & McMahon, F. J. (2010). The International Consortium on Lithium Genetics (ConLiGen): an initiative by the NIMH and IGSLI to study the genetic basis of response to lithium treatment. Neuropsychobiology, 62(1), 72-78.

## Claims

1. Method for generating a non-human animal model of a mental disorder comprising implanting human olfactory neural progenitors into a neurogenic niche of a non-human recipient animal.

2. Method, according to claim 1, wherein the mental disorder is selected from: bipolar disorder, schizophrenia, psychotic disorders, major depressive disorder, autism and/or addictive disorders.

3. Method, according to any one of the previous claims, wherein the mental disorder is bipolar disorder.

4. Method, according to any of the previous claims, comprising the following steps:
a. culturing human olfactory neural progenitors, and
b. implanting human olfactory neural progenitors into a neurogenic niche of a non-human recipient animal.

5. Method, according to any one of the previous claims, **characterized in that** the recipient animal is a mammal, preferably a rodent or primate, more preferably a rat or a mouse.

6. Method, according to any one of the previous claims, **characterized in that** the recipient animal is an immunodeficient or immunosuppressed mouse, preferably an immunodeficient BALB/c nude mouse.

7. Non-human animal model obtained or obtainable by the method of any of the claims 1 to 6.

8. Non-human animal model **characterized in that** it comprises human olfactory neural progenitors, or human differentiated cells selected from glia cells and/or neurons derived from said neural precursors, in the brain.

9. Non-human animal model, according to any one of claims 7 or 8, **characterized in that** it displays a mental disorder phenotype.

10. Non-human animal model according to any one of claims 7 to 9, **characterized in that** it displays a phenotype of bipolar disorder, schizophrenia, psychotic disorders, major depressive disorder, autism and/or addictive disorders.

11. A method for screening candidate compounds useful in the treatment of a mental disorder, or for evaluating the efficacy of a preventive or therapeutic treatment against a mental disorder, comprising administering said candidate compound to the non-human animal model of claims 7 to 10, wherein a positive response to treatment by the non-human animal of claims 7 to 10 is indicative that the candidate compound could be used in the treatment of the mental disorder in humans or that the candidate compound is effective as a preventive or therapeutic treatment against the mental disorder in humans.

12. Use of the non-human animal model of claims 7 to 10 to study the pathophysiology or behavior associated with a mental disorder.

13. Use of human olfactory neural progenitors for the generation of a non-human animal model of a mental disorder.

14. Use according to any one of claims 12 or 13, or method according to claim 11, wherein the mental disorder is selected from bipolar disorder, schizophrenia, psychotic disorders, major depressive disorder, autism and/or addictive disorders.

15. Use according to any one of claims 12 to 14, or method according to claim 11 or 14, wherein the mental disorder is bipolar disorder.
